# EUROPÄISCHE PATENTSCHRIFT

(11) **EP 3 819 032 B1**
(45) Veröffentlichungstag und Bekanntmachung des Hinweises auf die Patenterteilung: **10.06.2026**
(21) Anmeldenummer: 20205885.5
(22) Anmeldetag: 05.11.2020
(51) Int. Cl.: B05B 7/00

(54) **BEREITSTELLUNGSSYSTEM ZUM AUFBRINGEN VON UNTERSCHIEDLICHEN, ANWENDUNGSFALLBEZOGENEN, TEMPORÄR WIRKENDEN OBERFLÄCHENFUNKTIONSBESCHICHTUNGEN**
PROVIDISION SYSTEM FOR APPLYING DIFFERENT, APPLICATION-RELATED, TEMPORARY FUNCTIONAL SURFACE COATINGS
SYSTÈME DE PRÉPARATION POUR APPLIQUER DIFFÉRENTS REVÊTEMENTS DE SURFACE AGISSANT TEMPORAIREMENT, EN FONCTION DE L'APPLICATION

(30) Priorität: 07.11.2019 DE 102019130085; 03.11.2020 DE 102020128833
(43) Veröffentlichungstag der Anmeldung: 12.05.2021
(73) Patentinhaber: Karau, Michael, 48565 Steinfurt (DE)
(72) Erfinder: Karau, Michael, 48565 Steinfurt (DE)
(74) Vertreter: Meissner Bolte Partnerschaft mbB

(56) Entgegenhaltungen:
- WO-A1-2015/032465
- DE-A1- 102004 038 017
- DE-A1- 102017 220 028

## Beschreibung

Die Erfindung betrifft ein Verfahren zum Betreiben eines Bearbeitungsmaschinen-Bereitstellungssystems zum Aufbringen von unterschiedlichen, anwendungsfallbezogenen, temporär wirkenden sprühfähigen flüssigen, verdunstenden Mitteln zum Reinigen durch Entfernen vom Fremdstoffen oder zum Kühlen von Oberflächen bei der Bearbeitung von Werkstücken, nämlich plattenförmigen Holz- oder holzhaltigen Materialien mittels Sprühen durch mehrere an der Bearbeitungsmaschine angebrachte pneumatische Zweistoffdüsen mit Düsenkörper, welche von einem Vorratsgefäß gespeist werden, welches mit den flüssigen, verdunstenden Mitteln zum Reinigen oder Kühlen gefüllt werden, sowie mit einer Druckgasquelle, welche an die Zweistoffdüsen angeschlossen wird, um die Mittel zum Reinigen oder Kühlen zu fördern und auf das jeweilige Werkstück aufzutragen, gemäß Anspruch 1.

Im Bereich von Bearbeitungsanlagen, insbesondere bei der Holzbearbeitung besteht eine große Vielfalt an zu bearbeitenden und zu verarbeitenden Werkstoffen, insbesondere Massivhölzer und Holzwerkstoffen, die Notwendigkeit entsprechend den Werkzeugen, den Bearbeitungsschritten und Verfahrensparametern Versuche durchzuführen, um eine optimale Bearbeitungstechnologie für den jeweiligen Einzelfall erstellen zu können.

In vielen Fällen wird nach der Methode Trial-and-Error gearbeitet mit dem Ergebnis, dass Werkstücke, aber auch Werkzeuge beschädigt werden und unbrauchbar sind mit der Folge entsprechend hoher Kosten. Erlangtes Fachwissen ist nur punktuell vorhanden und wird in manchen Fällen nicht oder nur unzureichend weitergegeben. Wertvolles Know-how geht dadurch unter Umständen verloren oder muss erneut mit entsprechendem Aufwand erarbeitet werden.

Um insbesondere bei der Beschichtung von Schmalseiten plattenförmiger Werkstücke mit einem Beschichtungsmaterial den Aufwand zu senken, wurde im Rahmen der DE 10 2017 122 701 A1 offenbart, eine Beschichtungsvorrichtung und ein Beschichtungsverfahren so weiter zu entwickeln, dass den gestiegenen Anforderungen an ein optisches Äußeres und einer hohen Wertigkeit der Verbindung zwischen Beschichtungsmaterial und Werkstück Rechnung getragen wird.

Die Beschichtungsvorrichtung umfasst im dortigen Fall ein Andruckelement zum Anbringen eines Beschichtungsmaterials an einer zu beschichtenden Oberfläche eines Werkstückes und einer Einrichtung zum Herbeiführen einer Relativbewegung zwischen dem Werkstück und dem Andruckelement. Darüber hinaus umfasst die Beschichtungsvorrichtung eine Erfassungseinrichtung zum Erfassen einer Oberflächeneigenschaft an der zu beschichtenden Oberfläche des Werkstückes und eine Einrichtung zum Einwirken auf das einzusetzende Haftmittel, zum Beispiel einen Kleber.

Gemäß einem dortigen Ausführungsbeispiel ist die Beschichtungsvorrichtung mit einer Dosiereinrichtung zum Ändern der durch das Auftragselement am Werkstück aufzubringenden Haftmittelmenge versehen, wobei die Dosiereinrichtung eingerichtet ist, um auf der Grundlage des Erfassungsergebnisses der Erfassungseinrichtung die Haftmittelmenge zu ändern. Beispielsweise kann ein bestimmtes Material eines Werkstückes eine höhere oder geringere Menge des Haftmittels erfordern und somit auf das einzusetzende Haftmittel eingewirkt werden.

Insofern ist es vorgesehen, dass die Erfassungseinrichtung einen Sensor zum Erfassen der Oberflächenrauigkeit aufweist. Somit kann ermittelt werden, ob beispielsweise im Falle einer relativ rauen Werkstückoberfläche eine größere Haftmittelmenge benötigt wird. Ebenso ist es möglich, dass die Erfassungseinrichtung einen Sensor zum Erfassen der Oberflächentemperatur umfasst, um zu bestimmen, ob das Werkstück eine geeignete Oberflächentemperatur aufweist.

Aus der DE 10 2016 219 371 A1 ist ein System bekannt, anhand dessen vorrichtungsbezogene Daten von Bearbeitungsanlagen, insbesondere von Holzbearbeitungsvorrichtungen, auf unkomplizierte und sichere Weise zwischen mindestens zwei Bedienern anhand einer Datenfernübertragung geteilt werden können.

Anhand eines Hauptrechners wird eine Datenbank erzeugt und mittels Datenfernübertragung bereitgestellt, so dass mindestens zwei Arbeitsrechner zweier unterschiedlicher Bearbeitungsvorrichtungen auf diese zugreifen können, wobei zumindest einer der beiden Arbeitsrechner vorrichtungsbezogene Daten in der Datenbank des Hauptrechners speichert, womit diese für den anderen der beiden Arbeitsrechner verfügbar werden.

Bei der Vorrichtung und dem Verfahren zum Beschichten eines Werkstückes gemäß DE 10 2017 205 208 A1 besteht das Werkstück bevorzugt zumindest abschnittsweise aus Holz, Holzwerkstoffen, Kunststoff oder dergleichen. Eine Aktivierungseinheit dient zum Aktivieren einer aktivierbaren Haftschicht, die zum Fügen des Beschichtungsmaterials und des Werkstückes vorgesehen ist.

Die Vorrichtung weist mindestens ein Messelement auf, das so eingerichtet ist, mindestens eine beim Betrieb der Vorrichtung auftretende Prozessgröße, insbesondere Regelgröße und/oder Störgröße zu messen.

Bei dem System zum Aufbringen von temporär wirkenden Oberflächenfunktionsbeschichtungen mittels Sprühen durch eine oder mehrere pneumatische Zweistoffdüsen mit Düsenkörper nach EP 3 315 209 B1 kommt eine sprühfähige Oberflächenfunktionsbeschichtungsflüssigkeit zum Einsatz.

Die eingesetzten Zweistoffdüsen arbeiten nach dem Prinzip einer Strahlpumpe oder Injektordüse und dienen dazu, die Oberflächenfunktionsbeschichtungsflüssigkeit zu fördern und durch Sprühen auf eine zu behandelnde Oberfläche aufzutragen.

Die eingesetzten Zweistoffdüsen weisen ein Minimalmengendüsenventil mit einer Stelleinrichtung auf, welche sich im Düsenkörper befinden. Die Stelleinrichtung kann ein auswechselbares Schraubeinsatzstück oder aber eine auswechselbare Nadel umfassen.

Im Stand der Technik sind weiterhin Lösungen zu finden, die auf ein Verfahren zum Kantenbeschichten einer Möbelplatte gerichtet sind, wobei es darum geht, die Qualität beim Aufbringen des Kantenbandes auf einer Möbelplatte zu verbessern.

Diesbezüglich kommen Trenn-, Reinigungs- und/oder Kühlmittel zum Einsatz. Es soll hierdurch erreicht werden, dass sich keine Leim- oder Kleberreste an bereits angeleimten Kantenbandansätzen so anhaften, dass eine Entfernung nur mit großem Aufwand möglich ist.

Neben der diesbezüglich relevanten DE 10 2005 024 005 A1 ist weiterhin vorgesehen, ein Trennmittel mit Hilfe ortsfester Sprühdüsen aufzubringen, um eine Verbrauchsminimierung des Trennmittels zu erreichen. Insofern sind in den Kantenheckbereichen der sich gegenüberliegenden beschichteten Kanten jeweils die vorerwähnten Sprühdüsen angeordnet.

Obwohl es bekannt ist, Trennmittel, zum Beispiel auf der Basis alkoholischer Flüssigkeiten zum Zweck der Verbesserung der Ausbildung angeleimter Kantenbänder von Möbelplatten einzusetzen und umfangreiche Erfahrungen beim diesbezüglichen Aufsprühen von Trennmitteln bestehen, hat es sich gezeigt, dass zum einen die eingesetzten Düsen einem nicht unerheblichen Verschleiß unterliegen mit der Folge, dass unter Umständen zu viel oder unzureichend verteiltes Trenn- oder Reinigungsmittel aufgebracht wird.

Weiterhin ist es bei modernen Bearbeitungsmaschinen bekannt, dass selbige hochkomplexe Anlagen darstellen, die den Vorteil besitzen, relativ schnell auf unterschiedliche Bearbeitungsvorgänge mit unterschiedlichen Werkstücken zu reagieren.

Ein Problem dabei ist jedoch die notwendige, ebenso schnelle und nach Möglichkeit kostengünstige Anpassung der einzusetzenden Oberflächenfunktionsbeschichtungen, die für die Bearbeitung entsprechender Werkstücke erforderlich sind.

Wenn wie beim Beispiel der bereits oben kurz umrissenen Kantenanleimverarbeitungsvorrichtungen das Werkstück aus geänderten Werkstoffen oder geänderter Oberfläche besteht und darüber hinaus geänderte und auch aus unterschiedlichen Werkstoffen bestehende Kantenbänder verarbeitet werden müssen, ergibt sich häufig die Notwendigkeit, die durch Sprühen aufgebrachten Beschichtungen an die geänderten Verhältnisse Werkstück/Werkzeug anzupassen.

Im bekannten Stand der Technik ist es unter diesem Aspekt notwendig, verteilt an der Bearbeitungsmaschine angebrachte Behältnisse zur Aufnahme von Oberflächenfunktionsbeschichtungen auszutauschen, zu entfernen bzw. die Füllung händisch zu erneuern mit vorheriger Entleerung bzw. einem Leerfahren der Anlage. Der Austausch der in den Behältnissen enthaltenen Oberflächenfunktionsbeschichtungsmaterialien bzw. -flüssigkeiten erfordert einen sehr großen Erfahrungsschatz und liegt oft nicht in dem Ausbildungs- und Erfahrungsbereich des Anlagenfahrers. Fehler beim Einsatz entsprechender Beschichtungsflüssigkeiten sind daher nicht unüblich und eher die Regel.

Die Folge ist in vielen Fällen eine Störung der Bearbeitungsvorrichtung oder Bearbeitungsanlage bzw. eine mindere Qualität, die sich in einer unzureichenden Qualität der Kantenfuge, einer Welligkeit des aufgebrachten Kantenbandes, einer unzureichenden Festigkeit des Kantenbandes bezogen auf die Haftung an der Möbelplatte und ähnliches zeigt.

Bei der DE 10 2004 038 017 A1 ist es Aufgabenstellung, eine Möglichkeit zu schaffen, mit einer einzigen Austrag- bzw. Spritzanlage miteinander an sich unverträgliche Farb- bzw. Lacksysteme, insbesondere wässrige Epoxysysteme und andere Polyurethansysteme verarbeiten zu können. Diesbezüglich wird ein zeitlich aufeinanderfolgender Austrag miteinander ansonsten unverträglicher Farb- oder Lacksysteme durchgeführt. Die entsprechenden technischen Einrichtungen müssen diesbezüglich mittels Reinigungsfluid ausgewaschen werden, um alle Reste eines vorherigen Farb- oder Lacksystems zu entfernen.

Aus der WO 2015/032465 A1 ist eine Einrichtung zum Bereitstellen eines Applikationsmaterials vorbekannt. Dort geht es insbesondere um die Beschichtung von Fahrzeugbauteilen. Dies sollen insbesondere Lacke, Wachse, Kleber, Antidröhnmassen, Unterbodenschutzmassen, Dichtmassen oder dergleichen sein. Konkret kommen Förderpumpen zum Einsatz, die in der Lage sind, pastöse oder hochviskose Materialien aufzutragen. Als Problem wird dort herausgestellt, dass Beschichtungsanlagen zyklisch arbeiten, das heißt, dass deren Betrieb regelmäßig über längere Zeit, insbesondere zu Reinigungszwecken, eingestellt wird. In diesen nicht aktiven Phasen sollen bestimmte Grundvorgänge aufrechterhalten werden, um ein Wiedereinschalten der Applikationseinheit oder der ganzen Anlage problemlos zu ermöglichen.

Insofern wird also ein Zusetzen von Leitungen vermieden. Verschiedene Anlageneinheiten dieses Standes der Technik sind jeweils einer steuerbaren Regeleinheit zugeordnet, so dass vorgegebene Arbeitsmodi eingestellt oder aufrechterhalten werden können. Zur Programmierung werden entsprechende digitale Steuersignale bereitgestellt. Weiterhin ist eine Sensorik vorhanden, die das Erreichen des gewünschten Zustandes meldet und an eine übergeordnete Steuerung zurücksendet. Hierdurch kann eine Umprogrammierung erfolgen. Zum Vermeiden von Verstopfungen entsprechender Leitungen ist dort vorgeschlagen, eine druckreduzierte Umwälzung von Applikationsmaterial vorzunehmen. Die vorgeschlagenen Einheiten sollen die Temperatur, die Zusammensetzung, das Mischen, das Entgasen und das Verteilen des Applikationsmaterials beeinflussen. Ein konkreter Sensor kann als Temperatursensor ausgeführt sein oder die Viskosität des Applikationsmaterials bestimmen. Gemäß dieser vorbekannten Lehre geht es immer um das Applikationsmaterial bzw. das Bearbeitungsmaterial und dessen chemische oder physikalische Eigenschaften, die erfasst oder beeinflusst werden sollen.

Aus dem Vorgenannten ist es daher Aufgabe der Erfindung, ein weiterentwickeltes Verfahren zum Betreiben eines Bearbeitungsmaschinen-Bereitstellungssystem zum Aufbringen von unterschiedlichen, anwendungsfallbezogenen, temporär wirkenden sprühfähigen flüssigen, verdunstenden Mitteln anzugeben, welches es ermöglicht, nicht nur den Einsatz entsprechender Beschichtungsmengen zu minimieren, sondern darüber hinaus zur Qualitätssicherung bei der Ausführung entsprechender Bearbeitungsvorgänge beizutragen.

Die Lösung der Aufgabe der Erfindung erfolgt durch ein Verfahren gemäß der Merkmalskombination nach Anspruch 1, wobei die Unteransprüche zweckmäßige Ausgestaltungen und Weiterbildungen darstellen.

Es wird demnach von einem Verfahren zum Betreiben eines Bearbeitungsmaschinen-Bereitstellungssystems zum Aufbringen von unterschiedlichen, anwendungsfallbezogenen, temporär wirkenden sprühfähigen flüssigen, verdunstenden Mitteln zum Reinigen durch Entfernen von Fremdstoffen oder zum Kühlen von Oberflächen bei der Bearbeitung von Werkstücken mittels Sprühen durch mehrere an der Bearbeitungsmaschine angebrachte pneumatische Zweistoffdüsen mit Düsenkörper ausgegangen.

Die Zweistoffdüsen werden von einem Vorratsgefäß gespeist, welches mit den entsprechenden sprühfähigen Mitteln gefüllt ist oder gefüllt werden kann. Weiterhin ist eine Druckgasquelle vorhanden, welche an die Zweistoffdüsen anschließbar ist, um die Mittel zu fördern und auf das jeweilige Werkstück und/oder die Bearbeitungsmaschine oder Teile hiervon aufzutragen.

Es geht um ein Verfahren zum Betreiben eines Bearbeitungsmaschinen-Bereitstellungssystem zum Aufbringen von zunächst flüssigen Mitteln im Zusammenhang mit der Bearbeitung von Holzmaterialien, nämlich plattenförmigen Holzmaterialien, hier insbesondere wiederum um die Beschichtung von Möbelplatten mit Kantenbändern, wobei durch die Mittel verschiedene technologisch wichtige Effekte erreicht werden sollen. Zum einen geht es um eine gezielte Reinigung der Oberflächen der miteinander zu verbindenden Werkstücke. Weiterhin wird durch das Aufbringen von Flüssigkeiten auf eine Seite eines Kantenbandes, bevorzugt die von der Möbelplatte abgewandten Seite, eine Kühlung durch Bildung einer Wärmesenke ermöglicht, eine optimale Viskosität einer aufgebrachten oder aufzubringenden Kleberschicht gewährleistet, Überhitzungen vermieden und Verformungen der Struktur- oder Sichtschichten des jeweiligen Kantenbandes verhindert.

Das Vorratsgefäß ist als ein Mischgefäß ausgebildet, welches mit einer Vielzahl von Komponentengefäßen gesteuert in Verbindung steht, derart, dass nach einer vorgegebenen, programmierbaren Folge im Sinne einer Programmwahl Inhaltsstoffe aus den Komponentengefäßen in das Mischgefäß überführbar sind, wobei die Zweistoffdüsen aus dem Mischgefäß versorgt werden und getrennt aktivierbar sind.

Je nach Programmwahl wird also in dem Mischgefäß das für den Einsatzfall optimierte Mittel bereitgestellt und gegebenenfalls nachgefördert.

Dadurch, dass erst kurz vor dem Einsatz, das heißt vor dem Sprühvorgang, die Mischung im Mischgefäß erfolgt, können auch ansonsten sich über einen längeren Lagerungszeitraum entmischende Flüssigkeiten kombiniert und entsprechend als Auftrags- und Beschichtungsmittel eingesetzt werden.

Die im Mischgefäß enthaltene Mischmenge wird dabei auf die Geschwindigkeit der Bearbeitungstechnologie und den Verbrauch an Mitteln abgestimmt und grundsätzlich minimiert gehalten, um bei geänderten Produktions-, Umwelt- und/oder Materialbedingungen durch geänderte Komponentenzuführung zügig reagieren zu können, ohne dass ein Verwerfen von Restmengen einer Vorratsmischung notwendig wird.

Das Mischgefäß kann dabei in unmittelbarer Nähe der entsprechenden Bearbeitungsvorrichtung angeordnet werden, wobei die Komponentengefäße räumlich entfernt angeordnet sein können und dort zum Beispiel einer bestimmten Temperierung oder aber auch geschützten Lagerung unterliegen. Es kann demnach die im Mischgefäß enthaltene Menge anderen Temperatur- und insbesondere Umweltbedingungen unterliegen, als die Verhältnisse am Ort der Komponentengefäße und der darin befindlichen Komponenten, insbesondere Flüssigkeiten.

Weiterhin ist erfindungsgemäß eine Sensorik vorgesehen, welche Parameter des jeweiligen Werkstückes, der Werkstückumgebung und/oder der Bearbeitungsmaschine erfasst, um eine Auswahl von Inhaltsstoffen und deren Mischungsverhältnis zu treffen und ein diesbezüglich gespeichertes oder erstelltes Programm aus einem Datenspeicher abzurufen.

Demnach kann also über die Sensorik ermittelt werden, ob sich beispielsweise aufgrund des Tagesganges die Temperatur am Werkstück verändert hat, mit der Folge geänderter Verdunstungsverhältnisse einer Flüssigkeit, die das Mittel bildet. Wird eine derartige Veränderung erkannt, erfolgt eine Änderung der Mischung unter Rückgriff auf gespeicherte Programmdaten, die zum Beispiel in Versuchen gewonnen wurden.

Der Datenspeicher kann hier insbesondere über eine Kommunikationsschnittstelle abgerufen werden.

Die Kommunikationsschnittstelle kann drahtgebunden, aber auch drahtlos realisiert sein.

In einer besonderen Ausführungsform ist die Kommunikationsschnittstelle über das World Wide Web realisiert und der Datenspeicher über eine Cloud-Anbindung ausführbar.

Die Sensorik erfasst die Umgebungstemperatur des Werkstückes, die Werkstücktemperatur, die Umgebungsluftfeuchtigkeit, die Temperatur der Inhaltsstoffe bzw. der Inhaltsstoffmischung und/oder die Temperatur und/oder die Viskosität eines Bearbeitungshilfsmittels, insbesondere eines Haftmittels, ausgebildet als Klebstoff und ermittelt hieraus Parameter zum Abruf geeigneter Mischprogramme.

Mindestens eine der bereits erwähnten Zweistoffdüsen zum Auftrag der Mittel ist auf oder in einem Gefäß zur Aufnahme des Bearbeitungshilfsstoffes vorgesehen. Durch entsprechenden Eintrag der hierfür besonders ausgeführten Mittel lässt sich das diesbezügliche Gefäß zur Aufnahme des Bearbeitungshilfsstoffes, zum Beispiel eines Klebstoffes, leicht reinigen bzw. wird ein Anhaften eingebrachter Bearbeitungshilfsstoffe reduziert wiederum mit der Folge einer leichteren Reinigung.

Die vorerwähnte Bearbeitungsmaschine dient zum Beschichten von Schmalseiten von Werkstücken, nämlich plattenförmigen Werkstücken aus Holz oder holzhaltigen Materialien. Die Beschichtung erfolgt hier mittels sogenannten Kantenbändern und die Mittel dienen dem Reinigen, Kühlen und/oder Benetzen vor, während und nach dem Ausführen des Kantenfixierens, insbesondere Kantenanleimens.

Erfindungsgemäß ist darüber hinaus eine weitere Sensorik zur Qualitätskontrolle vorhanden. Diese Sensorik kann insbesondere als optoelektronischer Sensor, insbesondere wiederum als Kamera, realisiert werden, um zu überprüfen, in welcher Qualität die Beschichtungen ausgebildet wurden, wobei anhand erkannter Qualitätsparameter bzw. Qualitätsschwankungen eine geänderte Programmwahl für die Mischung der Mittel erfolgt. Ein Qualitätskriterium ist hier beispielsweise die verbleibende Breite einer Klebefuge, die Welligkeit des Kantenbandes, ein möglicherweise vorhandener Überstand des Kantenbandes vor dem Abfräsen desselben, eine abweichende Farbgestaltung oder dergleichen.

Durch kontinuierliche Auswertung diesbezüglicher, optisch erkennbarer und damit visualisierbarer Qualitätskriterien besteht die Möglichkeit der Ausführung eines Lernprozesses im Sinne einer Maschinensteuerung unter Berücksichtigung von Aspekten der künstlichen Intelligenz.

Das erfindungsgemäße Verfahren zum Betreiben des Bearbeitungsmaschinen-Bereitstellungssystem soll anhand einer Figur näher erläutert werden.

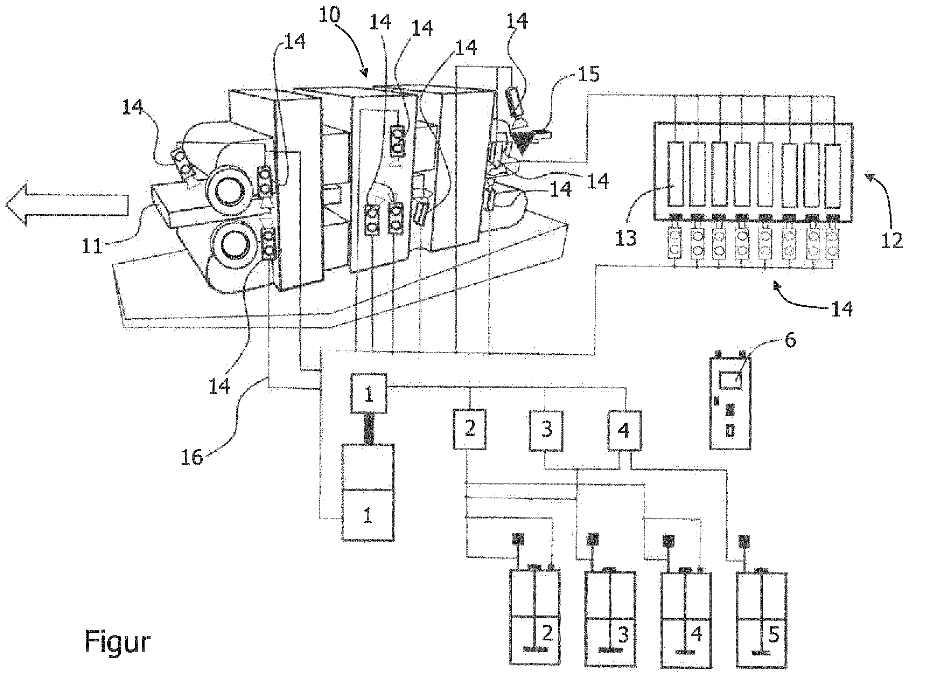

Die Figur zeigt hierbei eine prinzipielle Anordnung einer Bearbeitungsmaschine für plattenförmige Werkstücke, insbesondere eine sogenannte Kantenanleimmaschine 10, mit deren Hilfe Möbelplatten 11 an ihren Seitenflächen mit Kantenbändern 13 unterschiedlicher Art, Ausführung oder Qualität beschichtet werden.

In der Kantenanleimmaschine 10 durchläuft das zu bearbeitende Werkstück, das heißt die Möbelplatte 11, mehrere Stationen, wobei ausgangsseitig bezogen auf die durch einen Pfeil symbolisierte Laufrichtung des Werkstückes das entsprechende Kantenband, aus einem Kantenmagazin 12 stammend, bereits insbesondere durch Kleben fixiert ist.

Eine Vielzahl von Zweistoffdüsen 14 ist an unterschiedlichen Stationen im Prozess des Kantenanleimens angebracht.

So sind bereits dem Kantenbandmagazin 12 Zweistoffdüsen 14 zugeordnet, um eine Reinigung aufzubringender Kantenbänder 13 vor dem Zuführen zu bezwecken oder aber beispielsweise ein Antihaftmittel auf einer Oberflächenseite aufzubringen oder aber auf der Beschichtungsseite einen Klebeaktivator aufzutragen.

Weitere Düsen 14 befinden sich an oder in der Nähe eines Gefäßes 15 zur Aufnahme eines Haftmittels, insbesondere des bereits erwähnten Klebers. Durch entsprechendes Besprühen dieses Gefäßes wird ein unerwünschtes Anhaften des Klebstoffes im Gefäß verhindert und damit eine leichtere Reinigung ermöglicht.

Weitere, symbolhaft dargestellte Zweistoffdüsen 14 dienen dem Kühlen von Abschnitten der zu bearbeitenden Möbelplatte bzw. des auftragenden Kantenbandes, dem Entfernen von Fremdstoffen, das heißt dem Reinigen, dem Auftragen eines Glanzmittels oder dergleichen.

Eine Vielzahl von Komponentengefäßen 2; 3; 4; 5, die spezifischen Inhaltsstoffe aufnehmen, ist über einen Mischregler mit einem Mischgefäß 1 verbunden.

Das Mischgefäß 1 wiederum steht mit Zuleitungen 16 zu den Zweistoffdüsen 14 in Verbindung.

Über eine angedeutete Steuerung 6 können Mischprogramme abgerufen und jeweils im Mischgefäß 1 eine auf die entsprechende Bearbeitungstechnologie, die Werkstücke oder die Werkzeuge angepasste Mischung bereitgestellt werden.

Das vorgestellte Mischsystem ermöglicht es, sowohl wässrige Formulierungen bereitzustellen als auch flüchtige organische Verbindungen (VOC) zu reduzieren.

## Patentansprüche

1. Verfahren zum Betreiben eines Bearbeitungsmaschinen-Bereitstellungssystems zum Aufbringen von unterschiedlichen, anwendungsfallbezogenen, temporär wirkenden sprühfähigen flüssigen, verdunstenden Mitteln zum Reinigen durch Entfernen vom Fremdstoffen oder zum Kühlen von Oberflächen bei der Bearbeitung von Werkstücken, nämlich plattenförmigen Holz- oder holzhaltigen Materialien (11) mittels Sprühen durch mehrere an der Bearbeitungsmaschine angebrachte pneumatische Zweistoffdüsen (14) mit Düsenkörper, welche von einem Vorratsgefäß gespeist werden, welches mit den flüssigen, verdunstenden Mitteln zum Reinigen oder Kühlen gefüllt werden, sowie mit einer Druckgasquelle, welche an die Zweistoffdüsen (14) angeschlossen wird, um die Mittel zum Reinigen oder Kühlen zu fördern und auf das jeweilige Werkstück (11) aufzutragen,
wobei
das Vorratsgefäß ein Mischgefäß (1) ist, welches mit einer Vielzahl von Komponentengefäßen (2;3;4;5) gesteuert in Verbindung gebracht wird, um nach einer vorgegebenen Programmwahl Inhaltsstoffe aus den Komponentengefäßen (2;3;4;5) in das Mischgefäß (1) zu überführen, wobei die Zweistoffdüsen (14) aus dem Mischgefäß (1) versorgt werden und getrennt aktivierbar sind sowie mittels einer Sensorik die Umgebungstemperatur des Werkstückes, die Werkstücktemperatur, die Umgebungsluftfeuchtigkeit, die Temperatur der Inhaltsstoffe oder der Inhaltsstoffmischung und/oder die Temperatur und/oder die Viskosität eines Bearbeitungshilfsmittels in Form eines Haftmittels erfasst wird, um eine Auswahl von Inhaltsstoffen und deren Mischungsverhältnis zu treffen und ein diesbezüglich gespeichertes oder erstelltes Programm aus einem Datenspeicher (6) des Bearbeitungsmaschinen-Bereitstellungssystems abzurufen, wobei weiterhin mittels einer weiteren Sensorik zur Qualitätskontrolle anhand erkannter Qualitätsparameter oder Qualitätsschwankungen eine geänderte Programmwahl für die Mischung der flüssigen, verdunstenden Mittel zum Reinigen oder Kühlen erfolgt.

2. Verfahren nach Anspruch 1,
**dadurch gekennzeichnet, dass**
der Datenspeicher (6) über eine Kommunikationsschnittstelle abrufbar ist.

3. Verfahren nach Anspruch 2,
**dadurch gekennzeichnet, dass**
die Kommunikationsschnittstelle über das World Wide Web realisiert und der Datenspeicher (6) als Cloud-Anbindung ausgeführt wird.

4. Verfahren nach einem der vorangegangenen Ansprüche,
**dadurch gekennzeichnet, dass**
die Bearbeitungsmaschine (10) zum Beschichten von Schmalseiten der plattenförmigen Werkstücke (11) aus Holz oder holzhaltigen Materialien eingesetzt wird, wobei die Beschichtung mittels Kantenbändern (13) erfolgt und die flüssigen, verdunstenden Mittel für das Reinigen oder Kühlen vor oder während des Beschichtens modifiziert und jeweils entsprechend gemischt bereitgestellt werden.

5. Verfahren nach einem der vorangegangenen Ansprüche,
**dadurch gekennzeichnet, dass**
das Mischgefäß (1) in räumlicher Nähe und die Komponentengefäße (2;3;4;5) räumlich entfernt von der Bearbeitungsmaschine (10)angeordnet werden.

## Claims

1. A method for operating a processing-machine supply system for applying different, application-specific, temporarily effective sprayable liquid, evaporating agents for cleaning by removing contaminants or for cooling surfaces during the machining of workpieces, namely panel-shaped wood or wood-based materials (11), by spraying via a plurality of pneumatic two-fluid nozzles (14) having a nozzle body and mounted on the processing machine, which are supplied from a reservoir filled with the liquid, evaporating agents for cleaning or cooling, and with a compressed-gas source which is connected to the two-fluid nozzles (14) in order to convey the agents for cleaning or cooling and to apply them to the respective workpiece (11), wherein
the reservoir is a mixing vessel (1) which is controllably brought into communication with a plurality of component vessels (2; 3; 4; 5), in order to transfer constituents from the component vessels (2; 3; 4; 5) into the mixing vessel (1) following a predefined programme selection, wherein the two-fluid nozzles (14) are supplied from the mixing vessel (1) and can be activated separately and wherein, by means of sensors, the ambient temperature of the workpiece, the workpiece temperature, the ambient air humidity, the temperature of the constituents or of the mixture of constituents and/or the temperature and/or the viscosity of a processing aid in the form of an adhesive are detected, in order to select constituents and their mixing ratio and to retrieve a corresponding stored or generated programme from a data memory (6) of the processing-machine supply system, wherein, furthermore, by means of further sensors for quality control, on the basis of detected quality parameters or quality fluctuations, a modified programme selection is carried out for the mixture of the liquid, evaporating agents for cleaning or cooling.

2. The method according to claim 1, **characterized in that**
the data memory (6) can be accessed via a communication interface.

3. The method according to claim 2, **characterized in that**
the communication interface is implemented via the World Wide Web and the data memory (6) is configured as a cloud connection.

4. The method according to any one of the preceding claims, **characterized in that**
the processing machine (10) is used for coating narrow sides of the panel-shaped workpieces (11) made of wood or wood-based materials, wherein the coating is carried out by means of edge bands (13) and the liquid evaporating agents for cleaning or cooling are modified and each correspondingly mixed and provided before or during the coating process.

5. The method according to any one of the preceding claims, **characterized in that**
the mixing vessel (1) is arranged in spatial proximity to the processing machine and the component vessels (2; 3; 4; 5) are arranged spatially remote from the processing machine (10).

## Revendications

1. Procédé de fonctionnement d'un système d'alimentation d'une machine de traitement pour l'application de différents agents d'évaporation liquides pulvérisables à action temporaire, spécifiques à une application pour le nettoyage par élimination de corps étrangers ou pour le refroidissement des surfaces lors du traitement de pièces à usiner, à savoir des matériaux en feuilles en bois ou en matériaux dérivés du bois (11), par pulvérisation à travers plusieurs buses pneumatiques bi-composantes (14) munies de corps de buses, fixées à la machine de traitement, qui sont alimentées par un réservoir, qui contient les agents d'évaporation liquides pour le nettoyage ou le refroidissement, ainsi que par une source de gaz sous pression, qui est reliée aux buses bi-composantes (14) pour acheminer les agents de nettoyage ou de refroidissement et les appliquer sur la pièce à usiner (11), dans lequel
le réservoir est une cuve de mélange (1), qui est reliée de manière contrôlée à plusieurs cuves de composants (2 ; 3 ; 4 ; 5), afin de transférer les ingrédients des cuves de composants (2 ; 3 ; 4 ; 5) vers la cuve de mélange (1) selon un programme prédéfini, dans lequel les buses bi-composantes (14) sont alimentées par la cuve de mélange (1) et peuvent être activées séparément et des capteurs détectent la température ambiante de la pièce à usiner, la température de pièce à usiner, l'humidité ambiante, la température des ingrédients ou du mélange d'ingrédients et/ou la température et/ou la viscosité d'un adjuvant de traitement sous forme d'adhésif, afin de permettre une sélection des ingrédients et de leur proportion de mélange, ainsi que la récupération d'un programme enregistré ou créé à partir d'une mémoire de données (6) du système d'alimentation de machine de traitement, dans lequel en outre, des capteurs supplémentaires de contrôle qualité, sur la base des paramètres de qualité détectés ou des variations de qualité, déclenchent une sélection d'un programme modifié pour le mélange des agents d'évaporation liquides pour le nettoyage ou le refroidissement.

2. Procédé selon la revendication 1, **caractérisé en ce que**
la mémoire de données (6) est accessible via une interface de communication.

3. Procédé selon la revendication 2, **caractérisé en ce que**
l'interface de communication est mise en œuvre via le Web et la mémoire de données (6) est mise en œuvre sous forme de connexion au cloud.

4. Procédé selon une quelconque des revendications précédentes, **caractérisé en ce que**
la machine de traitement (10) est utilisée pour le revêtement des bords étroits de pièces à usiner en feuilles (11) en bois ou en matériaux dérivés du bois, dans lequel le revêtement est réalisé par placage de chants (13) et les agents d'évaporation liquides pour le nettoyage ou le refroidissement sont utilisés et modifiés avant ou pendant le processus de revêtement, puis mélangés en conséquence.

5. Procédé selon une quelconque des revendications précédentes, **caractérisé en ce que**
la cuve de mélange (1) est disposée à proximité immédiate de la machine de traitement (10), tandis que les cuves de composants (2 ; 3 ; 4 ; 5) sont disposées à distance de la machine de traitement (10).
